(19) Europäisches Patentamt
European Patent Office
Office européen des brevets

(11) **EP 1 688 407 B1**

(12) **EUROPEAN PATENT SPECIFICATION**

(45) Date of publication and mention of the grant of the patent:
**25.01.2017 Bulletin 2017/04**

(51) Int Cl.:
*C07C 51/44* *(2006.01)*      *C07C 51/50* *(2006.01)*
*C07C 57/04* *(2006.01)*

(21) Application number: **04787735.2**

(22) Date of filing: **08.09.2004**

(86) International application number:
**PCT/JP2004/013052**

(87) International publication number:
**WO 2005/051883 (09.06.2005 Gazette 2005/23)**

(54) **METHOD OF PURIFYING (METH)ACRYLIC ACID**

VERFAHREN ZUR AUFREINIGUNG VON (METH)ACRYLSÄURE

PROCEDE DE PURIFICATION D'ACIDE (METH)ACRYLIQUE

(84) Designated Contracting States:
**ES IT**

(30) Priority: **28.11.2003 JP 2003399124**

(43) Date of publication of application:
**09.08.2006 Bulletin 2006/32**

(73) Proprietor: **Mitsubishi Chemical Corporation
Chiyoda-ku
Tokyo 100-8251 (JP)**

(72) Inventors:
- **YADA, Shuhei,
  c/o Mitsubishi Chemical Corporation
  Minato-ku Tokyo 108-0014 (JP)**
- **OGAWA, Yasushi,
  c/o Mitsubishi Chem. Corporation
  Yokkaichi-shi,
  Mie 5108530 (JP)**
- **TAKASAKI, Kenji,
  c/o Mitsubishi Chemical Corp.
  Yokkaichi-shi,
  Mie 5108530 (JP)**

- **SUZUKI, Yoshiro,
  c/o Mitsubishi Chemical Corp.
  Yokkaichi-shi,
  Mie 5108530 (JP)**

(74) Representative: **Hoffmann Eitle
Patent- und Rechtsanwälte PartmbB
Arabellastraße 30
81925 München (DE)**

(56) References cited:
**WO-A1-03/043969      DE-A1- 3 837 955
JP-A- 10 087 551      JP-A- 49 018 820
JP-A- 49 085 016      JP-A- 2003 073 328
JP-A- 2003 342 231**

- **DATABASE WPI Week 200131 Derwent
  Publications Ltd., London, GB; AN 2001-293790
  XP002407820 & JP 2000 344688 A (MITSUBISHI
  CHEM CORP) 12 December 2000 (2000-12-12)**

EP 1 688 407 B1

## Description

### Technical Field

The present invention relates to a method of purifying (meth)acrylic acid, and more specifically to a method of purifying through distillation (meth)acrylic acid obtained through a vapor-phase catalytic oxidation process.

### Background Art

A method of producing (meth)acrylic acid may involve hydrolysis of a corresponding nitrile compound, but nowadays, the method mainly involves a vapor-phase catalytic oxidation process of a corresponding hydrocarbon, that is, propylene or isobutylene. A vapor-phase catalytic oxidation process, using a corresponding alkane which is more inexpensive as a raw material in place of an olefin, has been studied recently, too.

In production of (meth)acrylic acid through a vapor-phase catalytic oxidation process, a reaction product gas containing produced (meth)acrylic acid is brought into contact with an absorbing solvent such as water, to thereby recover (meth)acrylic acid in a gas as a (meth)acrylic acid solution. The solution contains, in addition to (meth)acrylic acid, various impurities produced during the vapor-phase catalytic oxidation as by-products including: carboxylic acids such as formic acid, acetic acid, maleic acid, and maleic anhydride; aldehydes such as acrolein, furfural, and benzaldehyde; and acetone, in production of acrylic acid.

Many methods of recovering purified (meth)acrylic acid from the (meth)acrylic solution have been proposed, and a method mainly used involves: removing the absorbing solvent and part of the impurities from the (meth)acrylic acid solution in a pre-purification step, to obtain crude (meth)acrylic acid substantially consisting of (meth)acrylic acid, a dimer thereof, and other heavy components; and purifying the crude (meth)acrylic acid in a purification step, to obtain a product having a desired quality.

A demand of acrylic acid, for example, has increased recently as a raw material for super absorbent polymers used in disposable diapers and the like and for food additives. High purity acrylic acid is demanded in such applications. Use of the crude acrylic acid as a raw material for an acrylic acid polymer of the super absorbent polymers and the like may result in problems including a slow polymerization reaction, reduction in degree of polymerization, and coloring of a polymerized product.

Thus, (meth)acrylic acid is purified through distillation industrially. A distillation method is generally used as a purification method for the crude (meth)acrylic acid obtained through vapor-phase catalytic oxidation. However, (meth)acrylic acid polymerizes very easily, and handling thereof causes problems.

For example, production of high purity acrylic acid through distillation conventionally had a problem of polymerization in a distillation column which is in high temperatures. Various developments and improvements have been made on items such as:

1) a kind of a polymerization inhibitor;
2) a concentration of and an introduction method for the polymerization inhibitor; and
3) operating conditions of a distillation column.

A method of producing high purity acrylic acid by separating and removing impurities from crude acrylic acid obtained through vapor-phase catalytic oxidation known as such conventional art involves distillation in the presence of hydrazines, for example (see JP 49-030312 A and JP 58-037290 B, for example). Further, another method involves distillation in the presence of hydrazine and ammonia (see JP 07-330659 A, for example). The method is presumably effective for removal of maleic acids. A known method of continuously producing high purity acrylic acid by preventing formation of a sludge in a distillation column involves using crude acrylic acid having a concentration of maleic acids of 2,000 ppm or less as a raw material for high purity acrylic acid (see JP 2001-316326 A, for example).

Further, when high purity acrylic acid is distilled through such techniques, acrylic acid tends to polymerize more easily with increasing purity, and an industrial technique for stable production of high purity acrylic acid has been demanded. As such a technique, a known technique for preventing polymerization inside a distillation column, for example, involves an attempt at suppressing polymerization of an easily polymerizable compound by adding copper dithiocarbamate (see JP 07-228548 A, for example). Further, another known technique involves supplying air (oxygen) into a distillation column for preventing polymerization of a vinyl group-containing compound such as acrylic acid or the like in a distillation step (see JP 2001-122909, for example).

As a result, the polymerization of (meth)acrylic acid in the distillation column has been suppressed, but other problems regarding polymerization have arisen in an operation of a distillation column. Such a problem in purification of (meth)acrylic acid involves the following. (Meth)acrylic acid having higher purity is received in a reflux tank for receiving a condensate of (meth)acrylic acid to cause formation of a polymerized product of (meth)acrylic acid regardless of a low temperature environment as a reflux tank of the distillation column, thereby possibly stopping a pump attached to the reflux tank due to the polymerizable product. Stopping of the pump inevitably stops the operation of the distillation column, and a method of suppressing the formation of the polymerized product has been desired.

JP 49 085016 A describes a method for inhibiting the polymerization of acrylic acid or acrylic esters during the distillation for separating or purifying the acrylic acid obtained by the vapor phase catalytic oxidation of propylene or acrolein, or the acrylic esters derived from said acrylic acid, wherein the method comprises carrying out the distillation operation in the presence of a polymerization inhibitor consisting of (A) at least one compound selected from the group consisting of hydroquinone, hydroquinone monomethyl ether, cresols, phenols, t-butyl catechol, diphenylamine, phenothiazine and methylene blue and (B) at least one compound selected from the group consisting of copper dimethyldithiocarbamate, copper diethyldithiocarbamate, copper dibutyldithiocarbamate and copper salicylate, and a molecular oxygen-containing gas.

Disclosure of the Invention

An object of the present invention is to provide a method of efficiently producing high purity (meth) acrylic acid by preventing formation of a polymerized product of (meth) acrylic acid in a condensate obtained in production of high purity (meth)acrylic acid through distillation.

That is, the inventors of the present invention have studied intensively to solve the conventional problems in continuous industrial production of high purity acrylic acid, and the studies have confirmed the following.

(1) Oxygen (usually air) supplied to a distillation column for acrylic acid for inhibiting polymerization is consumed inside the column and does not provide a polymerization inhibition effect to an acrylic acid liquid in a reflux tank.

(2) Thus, a method of increasing oxygen (usually air) supplied to the column is studied. However, the distillation column is at high temperatures. Thus, there may be formed a detonating gas in the distillation column if a large volume of air is supplied into the distillation column. Thus, the supply of oxygen cannot be increased.

(3) Further, the distillation column for acrylic acid is usually a vacuum distillation column for reducing a temperature inside the distillation column for preventing polymerization. Thus, simple increase of air causes increase in a flow rate of a gas in the distillation column which results in: reduction of production efficiency by exceeding of a limit of tray performance and increase of an inert gas (that is, air) in a gas supplied to a column top condenser, in an existing distillation column; and an uneconomical operation by increase of a diameter of the distillation column and increase of a heat transfer area of the column top condenser, in a new distillation column. Further, the inventors of the present invention have found out an industrially simple and effective method as follows.

(4) New supply of oxygen (usually air) into the acrylic acid liquid in the reflux tank can avoid formation of the polymerizable compound, for a stable operation of a pump attached to the reflux tank over a long period of time.

That is, that present invention relates to a method of purifying (meth) acrylic acid, comprising the steps of: distilling a crude (meth)acrylic acid-containing liquid comprising acrylic acid or methacrylic acid to obtain a condensate of (meth)acrylic acid having a (meth)acrylic acid purity of 90% or more by a distillation apparatus provided with a reflux tank; and supplying an oxygen-containing gas comprising oxygen to the condensate of (meth)acrylic acid in the reflux tank for receiving the condensate of (meth)acrylic acid.

In the present invention, (meth)acrylic acid refers to acrylic acid or methacrylic acid.

Brief Description of the Drawings

Fig. 1 is a vertical sectional view showing an example of a reflux tank used in an embodiment of the present invention.
Fig. 2 is a vertical sectional view showing another example of a reflux tank used in an embodiment of the present invention.
Fig. 3 is a vertical sectional view showing a still another example of a reflux tank used in an embodiment of the present invention.
Fig. 4 is a vertical sectional view showing a yet another example of a reflux tank used in an embodiment of the present invention.
Fig. 5 is a schematic diagram showing an example of a distillation apparatus for acrylic acid that can be used in an embodiment of the present invention.
Fig. 6 is a schematic diagram showing another example of a distillation apparatus for acrylic acid that can be used in an embodiment of the present invention.

Best Mode for carrying out the Invention

Hereinafter, the present invention will be described in more detail.

A method of purifying (meth)acrylic acid of the present invention comprises the steps of: distilling a crude (meth)acrylic acid-containing liquid comprising acrylic acid or methacrylic acid to obtain a condensate of (meth) acrylic acid having a (meth) acrylic acid purity of 90% or more (hereinafter, the step may also be referred to as a "distillation step") by a distillation apparatus provided with a reflux tank; and supplying an oxygen-containing gas comprising oxygen into the condensate of (meth) acrylic acid in the reflux tank for receiving the condensate of (meth)acrylic acid (hereinafter, the step may also be referred to as an "oxygen supply step").

The distillation step is not particularly limited so long as the step is capable of obtaining a condensate of (meth)acrylic acid having a purity of 90% or more, preferably 95% or more. The known techniques described above in the section titled Background Art, for example, can be used for such a distillation step.

The crude (meth)acrylic acid-containing liquid is not particularly limited so long as the liquid containing acrylic acid or methacrylic acid. Examples of such a (meth)acrylic acid-containing liquid include (meth)acrylic acid itself, and a solution of the (meth) acrylic acid in an appropriate solvent such as water and an organic solvent. The crude (meth)acrylic acid-containing liquid is preferably obtained through vapor-phase catalytic oxidation in terms of productivity and the like. The crude (meth)acrylic acid-containing liquid can be obtained through the known techniques described above in the section titled Background Art, for example.

A method of distilling used in the distillation step is not particularly limited so long as the method of distilling which provides a condensate of (meth)acrylic acid having the above-mentioned purity from the (meth)acrylic acid-containing liquid. Such a method of distilling may employ various methods of distilling such as simple distillation and precision distillation. The distillation may be applied to a continuous type or a batch type. The crude (meth)acrylic acid-containing liquid is preferably distilled using a distillation column as usual in terms of industrial production and the like. A purity of a condensate can be adjusted depending on various conditions such as a distillation column used, a type of packing, a reflux ratio, a distillation temperature, and a kind or amount of an additive added to a crude (meth)acrylic-containing liquid.

In the distillation step, the crude (meth)acrylic acid-containing liquid is preferably distilled in the presence of a known polymerization inhibitor or polymerization retarder (hereinafter, may be simply referred to as a "polymerization inhibitor") such as a phenol compound or a phenothiazine compound, for preventing formation of a polymerized product of (meth)acrylic acid and for stably obtaining high purity (meth)acrylic acid over a long period of time.

A method of obtaining high purity crude (meth)acrylic acid through the production of crude (meth)acrylic acid will be described with acrylic acid as an example. The method of obtaining high purity crude (meth)acrylic acid through the production of crude (meth)acrylic acid include the following (1) to (3).

(1) A method includes: an oxidation step for producing acrylic acid through vapor-phase catalytic oxidation of propane, propylene, and/or acrolein; a collecting step for collecting acrylic acid as an aqueous solution of acrylic acid by bringing a gas containing acrylic acid formed in the oxidation step into contact with water; an extraction step for extracting acrylic acid by using an appropriate extracting solvent from the aqueous solution of acrylic acid obtained in the collecting step; a separation step for separating the acrylic acid and the solvent from the obtained extracted liquid; a low boiling point component removal step for removing a low boiling point component from the separated acrylic acid; a purification step for purifying an acrylic acid-containing liquid from which the low boiling point component was removed through distillation or the like; a recovery step for recovering valuable substances by supplying to a decomposition reaction column as a raw material, a high boiling point liquid (liquid high boiling point substance) containing Michael adducts of acrylic acid recovered from the above-mentioned steps and a polymerization inhibitor used in each of the steps; and a recycle step for supplying the recovered valuable substances to any step after the collecting step.

(2) A method includes: an oxidation step for producing acrylic acid through vapor-phase catalytic oxidation of propane, propylene, and/or acrolein; a collecting step for collecting acrylic acid as an aqueous solution of acrylic acid by bringing a gas containing acrylic acid formed in the oxidation step into contact with water; an azeotropic separation step for taking out crude acrylic acid from a column bottom of an azeotropic separation column by distilling the aqueous solution of acrylic acid obtained in the collecting step in the azeotropic separation column in the presence of an azeotropic solvent; an acetic acid separation step for removing acetic acid from the acrylic acid taken out; a purification step for removing high boiling point impurities; a recovery step for recovering valuable substances by supplying to a decomposition reaction column as a raw material, a high boiling point liquid containing Michael adducts of acrylic acid recovered from the above-mentioned steps and a polymerization inhibitor used in each of the steps; and a recycle step for supplying the valuable substances to any step after the collecting step.

(3) A method includes: an oxidation step for producing acrylic acid through vapor-phase catalytic oxidation of propane, propylene, and/or acrolein; a collecting/separation step for collecting acrylic acid as an organic solution of acrylic acid by bringing a gas containing acrylic acid formed in the oxidation step into contact with an organic solvent and simultaneously removing water, acetic acid, and the like; a separation step for taking out the acrylic acid from the organic solution of acrylic acid; a recovery step for recovering valuable substances by supplying to a decomposition reaction column as a raw material, a high boiling point liquid containing a polymerization inhibitor and an organic solvent used in each of the steps and Michael adducts of acrylic acid recovered from the above-mentioned steps; a recycle step for supplying the valuable substances to any step after the collecting step; and a solvent purification step for purifying part or whole of the recovered organic solvent.

Crude acrylic acid used as a raw material for obtaining high purity acrylic acid in an industrial acrylic acid plant is generally a liquid obtained after removal of a low boiling point component. Examples of impurities include: low boiling point impurities remained slightly such as water, furfural, benzaldehyde, and acetic acid; and high boiling point impurities such as a (meth)acrylic acid dimer, a (meth)acrylic acid trimer, maleic anhydride, ß-hydroxypropinonic acid, and ß-alkoxypropionic acid. By distilling the liquid using a distillation column, acrylic acid having a purity of 90% or more can be obtained from a column top. Further, a condensate of (meth)acrylic acid can be also directly obtained through recovery of valuable substances in the recovery step.

The present invention can be applied to methacrylic acid in the same manner as in acrylic acid. When the present invention is applied to production of methacrylic acid, crude methacrylic acid is obtained through vapor-phase catalytic oxidation of isobutylene and/or t-butyl alcohol and citraconic acids in addition of aldehydes, ketones, and maleic acids as crude acrylic acid are contained as impurities in the crude methacrylic acid, for example.

A column top liquid obtained through distillation of a crude (meth)acrylic acid-containing liquid usually contains as trace impurities, carboxylic acids such as maleic acids and acetic acid, aldehydes such as furfural and benzaldehyde, and water. Thus, an additional step (removal of impurities (described below) by addition of hydrazines or mercaptans) is preferably carried out for obtaining acrylic acid having higher purity.

When a purified product is used as high purity acrylic acid, for example, for removing impurities which cannot be removed economically through distillation separation, a reactant reacting with such impurities for forming a compound which can be separated through distillation is added to a crude acrylic acid-containing liquid,; and the crude acrylic acid-containing liquid is supplied to a distillation column. Removal of an aldehyde component by treating (meth)acrylic acid containing the aldehyde component with an aldehyde remover is disclosed in JP 2001-058970 A or JP 2001-213839 A, and such techniques can be applied to the present invention.

For separation of the impurities through distillation, the crude (meth)acrylic acid-containing liquid preferably further contains a hydrazine compound. Examples of the hydrazine compound include hydrazine, hydrazine hydrate, phenylhydrazine, hydrazine sulfate, and hydrazine chloride. The hydrazine compound may be used alone or as a mixture of two or more thereof. An addition amount of the hydrazine compound is selected accordingly depending on an amount of impurities to be removed and an allowable concentration of impurities in high purity acrylic acid obtained after the distillation.

The hydrazine compound is preferably added as it is to the crude (meth)acrylic acid-containing liquid. An addition amount of the hydrazine compound is usually 0.1 to 2 times moles, preferably 0.5 to 2 times moles, more preferably 0. 5 to 1 time moles with respect to a total amount of aldehydes such as furfural and benzaldehyde and maleic acids in the crude (meth)acrylic acid-containing liquid.

A method of adding the hydrazine compound to the crude (meth)acrylic acid-containing liquid is not particularly limited so long as the hydrazine compound and the impurities to be removed can react with each other.

In present invention, distillation purification is preferably carried out after: adding the hydrazine compound to the crude (meth)acrylic acid-containing liquid before being supplied to the distillation column; and making the impurities such as maleic acids in the crude (meth)acrylic acid-containing liquid react with the hydrazine compound. A reaction between the hydrazine compound and the impurities is preferably carried out using a reaction device which can secure required temperature and residence time. Examples of the reaction device may include a reaction tank equipped with a stirrer and a tubular reaction tank. A reaction temperature is preferably as low as possible, and is specifically selected within a range from a melting point of acrylic acid or more to 50°C or less. A reaction time is preferably 10 minutes or more, and a residence time is usually about 30 minutes to 3 hours.

The reaction between the hydrazine compound and the impurities can be carried out in the distillation column. In this case, it is preferable that there is a residence time of 10 minutes to 5 hours preferably, 20 minutes to 3 hours more preferably between addition of the hydrazine compound to the crude (meth)acrylic acid compound and obtaining purified (meth) acrylic acid as a distillate from a column top of the distillation column. A short residence time may result in an insufficient reaction between the hydrazine compound and the impurities. Too long a residence time may result in increase of the impurities through a decomposition reaction of a reaction product. Thus, a residence time is selected within the above range.

The impurities to be removed such as maleic acids are removed from crude (meth)acrylic acid with the hydrazine compound added through distillation treatment.

For separation of the impurities through distillation, the crude (meth)acrylic acid-containing liquid preferably further contains a mercaptans. Examples of the mercaptans include n-butyl mercaptan, n-octyl mercaptan, and n-dodecyl mercaptan. In the present invention, the mercaptans may be used alone or as a mixture of two or more thereof.

When the mercaptans is used, an aldehyde component in the crude (meth)acrylic acid-containing liquid can be removed by passing the crude (meth)acrylic acid-containing liquid having the mercaptans added through a resin column packed with a sulfonic acid-type cation exchange resin at 20 to 90°C and LHSV (liquid hourly space velocity) = 0.1 to 10 hr$^{-1}$. The obtained liquid is used as a supply liquid to the distillation column. The liquid may be passed through the column as a downflow or an upflow. The mercaptans as an aldehyde remover is generally used in 1 to 8 times moles of the aldehyde component.

The oxygen supply step is not particularly limited so long as the oxygen-containing gas can be supplied to a condensate of (meth)acrylic acid in a reflux tank for receiving the condensate of (meth)acrylic acid. Such an oxygen supply step can employ a known technique involving gas-liquid contact by supplying a gas into a liquid.

The oxygen-containing gas is not particularly limited so long as the gas contains oxygen. Examples of such an oxygen-containing gas include a single gas of oxygen or air, and a mixed gas of the single gas and one or more gases selected from the following gases (1) to (4).

    (1) nitrogen
    (2) process exhaust gas
    (3) air (except when the single gas is air)
    (4) carbon dioxide

The process exhaust gas as used herein refers to a process exhaust gas discharged from the above-mentioned acrylic acid process or from an acrylate plant adjacent to the acrylic acid plant or the like, and is a gas component which does affect the place where high purity acrylic acid is used in the present invention. The process exhaust gas may be a gas component consisting of substances or compounds which do not affect the quality of high purity (meth)acrylic acid obtained in the present invention or a gas component containing the component in an amount which does not affect the quality. Specific examples of the process exhaust gas include "water" and "acetic acid".

An oxygen content of the oxygen-containing gas is not particularly limited so long as a detonating gas is not formed under operating conditions of a reflux tank, but is preferably 5 to 30 vol%. Air is particularly preferable as the oxygen-containing gas.

A supply of the oxygen-containing gas depends on a size of the reflux tank and a residence time of a condensate of (meth)acrylic acid in the reflux tank. However, a ratio ($Nm^3/t$) of a supply of oxygen in the oxygen-containing gas to a flow rate of the condensate into the reflux tank at normal condition (0°C, 101 kPa (1 atm)) preferably satisfies a relationship shown by the following equation.

$$0.004 \leq A / B \leq 1.0$$

(wherein, A denotes an $O_2$ supply ($Nm^3/h$), and B denotes a flow rate (t/h) of the condensate into the reflux tank. A symbol N in "$Nm^3/h$" indicates a value at normal condition (0°C, 101 kPa (1 atm): Normal.)

Further, in the present invention, a polymerization inhibitor (polymerization inhibitor and/or polymerization retarder) may be used for suppressing formation of a polymerized product in production or purification as described above. A known polymerization inhibitor and/or polymerization retarder may be used as a polymerization inhibitor, and specific examples thereof include a copper-based compound such as copper (meth)acrylate or a copper dithiocarbamate, a phenol compound, and a phenothiazine compound. The polymerization inhibitor may be used alone or as a mixture of a plurality thereof. The polymerization inhibitor may be added to the crude (meth)acrylic acid-containing liquid or to the condensate of (meth)acrylic acid. Either case is preferable for preventing formation of a polymerized product in purification of (meth)acrylic acid.

Examples of the phenol compound used in the present invention include hydroquinone, methoquinone (methoxyhydroquinone), pyrogallol, catechol, resorcin, phenol, and cresol. The phenol compound may be used alone, or as a mixture of two or more thereof. An addition amount of the phenol compound is 0 to 800 ppm by weight, preferably 50 to 600 ppm by weight with respect to the crude (meth)acrylic acid-containing liquid supplied to the distillation column. Further, an addition amount of the phenol compound is 0 to 500 ppm by weight, preferably 1 to 300 ppm by weight with respect to the condensate of (meth)acrylic acid supplied to the reflux tank. A small addition amount may result in an insufficient polymerization suppression effect. Too large an addition amount does not adversely affect the polymerization suppression effect but is not preferable economically.

Examples of the phenothiazine compound used in the present invention include phenothiazine, bis-(a-methylbenzyl)phenothiazine, 3,7-dioctylphenothiazine, and bis-(a-dimethylbenzyl)phenothiazine. The phenothiazine compound may be used alone or as a mixture of two or more thereof. An addition amount of the phenothiazine compound is 0 to 400 ppm by weight, preferably 50 to 300 ppm by weight with respect to (meth)acrylic acid supplied to the distillation column. Further, an addition amount of the phenothiazine compound is 0 to 200 ppm by weight, preferably 1 to 100 ppm by weight with respect to the condensate of (meth)acrylic acid supplied to the reflux tank. A small addition amount may result in an insufficient polymerization suppression effect. Too large an addition amount does not adversely affect the polymerization suppression effect but is not preferable economically.

Examples of the copper-based compound include copper compounds such as cupric chloride, copper acetate, copper carbonate, copper (meth)acrylate, copper dimethyldithiocarbamate, copper diethyldithiocarbamate, and copper dibutyldithiocarbamate. The polymerization inhibitor may be used alone or as a mixture of two or more thereof. An addition amount of the polymerization inhibitor is not particularly limited, but is preferably about 1 to 1,000 ppm.

Examples of the copper dithiocarbamate include: copper dialkyldithiocarbamates such as copper dimethyldithiocarbamate, copper diethyldithiocarbamate, copper dipropyldithiocarbamate, and copper dibutyldithiocarbamate; copper cycloalkylene dithiocarbamates such as copper ethylenedithiocarbamate, copper tetramethylenedithiocarbamate, copper pentamethylenedithiocarbamate, and copper hexamethylenedithiocarbamate; and copper cycloxydialkylene dithiocarbamates such as copper oxydiethylene dithiocarbamate. Such a copper dithiocarbamate may be used alone or as a mixture of two or more thereof.

An addition amount of the copper dithiocarbamate is 1 to 100 ppm by weight, preferably 10 to 80 ppm by weight with respect to the (meth)acrylic acid-containing liquid supplied to the distillation column. Further, an addition amount of the copper dithiocarbamate is 0 to 50 ppm by weight, preferably 1 to 20 ppm by weight with respect to the condensate of (meth)acrylic acid supplied to the reflux tank. A small addition amount may result in an insufficient polymerization suppression effect. A large addition amount is not preferable because corrosion of an apparatus occurs at a column bottom of the distillation column. In a distillation system of the present invention, the copper dithiocarbamate presumably has a larger effect of polymerization suppression on a column bottom liquid compared to on a liquid flowing down inside the distillation column. Thus, the copper dithiocarbamate is preferably added to the crude (meth) acrylic acid-containing liquid as a raw material or to the column bottom liquid of the distillation column.

Copper (meth)acrylate used in the present invention functions as a polymerization inhibitor for (meth)acrylic acid similar to the copper dithiocarbamate. A combination of both copper (meth)acrylate and the copper dithiocarbamate provides a significant effect on prevention of polymerization.

Copper (meth)acrylate can be prepared by dissolving copper carbonate, copper chloride, an organic acid salt of copper, copper hydroxide, or a copper powder in (meth)acrylic acid. Copper carbonate is particularly preferable. Copper chloride is not preferable because a distillation column for (meth)acrylic acid is generally constructed of a stainless steel material, and copper chloride may cause stress corrosion cracking. Specific examples of a substance dissolved in (meth)acrylic acid for formation of copper (meth)acrylate used in the present invention include: cupric carbonate as a carbonate; copper formate, copper acetate, or copper salicylate as an organic acid salt; and cuprous hydroxide or cupric hydroxide as a hydroxide. Further, a copper powder may be directly dissolved in (meth)acrylic acid. Such copper (meth)acrylate may be used alone or as a mixture of two or more thereof.

Copper (meth)acrylate may also be obtained by dissolving in an solvent containing (meth)acrylic acid. The solvent used in this case is preferably a solvent having a boiling point higher than that of (meth)acrylic acid to prevent contamination of high purity (meth)acrylic acid with the solvent, obtained from the column top of the distillation column. Specific examples of the solvent include diphenyl ether, o-phthalates, oleates, adipates, hydrocarbons distilled from middle oil, and heat-transfer oil having a boiling point of 170°C or more. The solvent may be used alone or as a mixed solvent of two or more thereof.

When the crude (meth)acrylic acid-containing liquid as a raw material to be distilled contains water, water can also be used as a solvent having a boiling point lower than that of (meth)acrylic acid. A water content can be determined considering an allowable value in high purity (meth)acrylic acid to be obtained and a required addition amount of copper (meth)acrylic acid. When the crude (meth) acrylic acid-containing liquid contains no water, an appropriate amount of water may be added. However, dehydration may be required depending on product specification of a purified product, and water must be added carefully.

An addition amount of copper (meth)acrylate is 1 to 100 ppm by weight, preferably 5 to 80 ppm by weight with respect to the crude (meth)acrylic acid-containing liquid supplied to the distillation column, assuming that all of dissolved copper converts into copper (meth)acrylate. Further, an addition amount of copper (meth)acrylate is 0 to 50 ppm by weight, preferably 1 to 20 ppm by weight with respect to the condensate of (meth)acrylic acid supplied to the reflux tank. A small addition amount may result in an insufficient polymerization suppression effect. Too large an addition amount is not preferable because corrosion of an apparatus occurs at a column bottom of the distillation column.

Copper (meth)acrylate has a large effect of on a liquid flowing down inside the distillation column unlike the copper dithiocarbamate. Thus, copper (meth)acrylate is preferably added to the crude (meth)acrylic acid-containing liquid as a raw material or the liquid (condensate) at the column top of the distillation column.

One or more polymerization inhibitors having different functions as described above is used in the present invention. Other polymerization inhibitors except the hydrazine compound, the copper dithiocarbamate, copper (meth)acrylate, the phenol compound, and/or the phenothiazine compound can be used as required.

Examples of the other polymerization inhibitors include: N-oxyl compounds such as t-butyl nitroxide, 2,2,6,6-tetramethyl-4-hydroxypiperidyl-1-oxyl, 2,2,6,6-tetramethylpiperidyl-1-oxyl, 2,2,6,6-tetramethylpiperidinoxyl, 4-hydroxy-2,2,6,6-tetramethylpiperidinoxyl, and 4,4',4"-tris-1-(2,2,6,6-tetramethylpiperidinoxyl)phosphite; phenylenediamines such as p-phenylenediamine; nitroso compounds such as N-nitrosodiphenylamine; ureas such as urea; and thioureas such as thiourea. The other polymerization inhibitor may be used alone or in combination of two or more thereof.

A method of adding copper (meth)acrylate, a copper dithiocarbamate, a phenol compound, and a phenothiazine compound each exerting a polymerization suppression effect is not particularly limited. Examples of the method include: a method involving directly adding the compound exerting a polymerization suppression effect to the crude (meth)acrylic acid-containing liquid supplied to the distillation column or to the condensate of the (meth)acrylic acid liquid refluxed as a distillate; and a method involving dissolving by using an appropriate solvent and adding the solution to the crude (meth)acrylic acid-containing liquid or the distillation column. An addition temperature may also be determined arbitrarily.

In the present invention, a substance other than those described above may be included depending on the steps , but a kind thereof is not particularly limited so long as the substance has a boiling point higher than that of (meth) acrylic acid of the condensate defined in the present invention.

In the present invention, the crude (meth)acrylic acid-containing liquid having the hydrazine compound, copper (meth)acrylate, and the copper dithiocarbamate added is subjected to distillation treatment, to thereby remove impurities to be removed. The applications of the obtained (meth)acrylic acid are not particularly limited, and the obtained (meth) acrylic acid may be used in various applications such as a raw material for (meth)acrylate, a raw material for high purity (meth)acrylic acid for super absorbent polymers, and general (meth)acrylic acid product.

The present invention prescribes that the oxygen supply step is carried out when a (meth)acrylic acid concentration is 90% or more in the condensate. However, the oxygen supply step can also be employed in a case where a (meth) acrylic acid concentration is less than 90% in the condensate depending on the situation. Examples of such a situation include: a case where a temperature of the condensate in a reflux tank is relatively high (40° C or more, for example); and a case where a substance which easily causes an exothermic reaction with (meth)acrylic acid exists in the condensate or a substance which easily polymerizes exists in the condensate in the reflux tank (acrolein, for example). By supplying the oxygen-containing gas to the condensate under the conditions of easily causing polymerization of (meth)acrylic acid as described above, high purity (meth) acrylic acid can be produced stably over a long period of time.

A method of purifying (meth)acrylic acid of the present invention may be carried out by using a usual apparatus or device used in production or purification of (meth)acrylic acid as it is or with partial modification thereof.

The distillation column is one generally used in a chemical plant. That is, examples of the distillation column include a perforated-plate column, a bubble cap column, a packed column, and combination thereof (a combination of a perforated-plate column and a packed column, for example). The presence or absence of an overflow weir or downcomer does not matter in the present invention. Either case can be used in the present invention.

Specific examples of trays include bubble cap trays each having a downcomer, perforated-plate trays, bubble trays, SUPERFRAC trays , MAX-FRAC trays , and dual flow trays without downcomers.

Examples of packing preferably used in the present invention include: conventionally used packing such as columnar packing, cylindrical packing, saddle packing, spherical packing, cubic packing, and pyramidal packing; and recently commercially available structured packing and random packing having a special shape as high performance packing.

Examples of commercially available structured packing include: gauze structured packing such as SULZER PACKING (available from Sulzer Brothers Ltd.), SUMITOMO-SULZER PACKING (available from Sumitomo Heavy Industries, Ltd.), and TECHNOPACK (available from MITSUI & CO., LTD.); sheet structured packing such as MELLAPAK (available from Sumitomo Heavy Industries, Ltd.), TECHNOPACK (available from MITSUI & CO. , LTD.), and MC PACK (available from Mitsubishi Chemical Engineering Corporation); grid structured packing such as FLEXI-GRID (available from Koch-Glitsch); and other structured packing such as GEM-PAK (available from Koch-Glitsch), MONTZ-PAK (available from Julius Montz GmbH), GOOD ROLL PACKING (available from Tokyo Tokushu Kanaami K. K.), HONEYCOMB PACK (available from NGK Insulators, Ltd.), and IMPULSE PACKING (available from Nagaoka International Corporation).

Examples of commercially available random packing include: Raschig ring, PALL RINGS (available from BASF Aktiengesellschaft); CASCADE MINI-RING (available from Mass Transfer Ltd.); IMTP (available from Saint-Gobain NorPro); INTALOX SADDLES (available from Saint-Gobain NorPro); TELLERETT (available from Nittetsu Chemical Engineering Ltd.); and FLEXI RINGS (available from JGC Corporation).

The packing may be used alone, or in combination of two or more thereof. Further, the packing may be used in combination with the trays conventionally used.

Specific operating conditions of the distillation column may be usually selected from a column bottom temperature of 60 to 100° C and a column top pressure of 1.33 to 26.7 kPa.

In the present invention, a column top gas cooling heat exchanger attached to the distillation column may be used as a condenser for condensing a vapor of (meth)acrylic acid obtained through distillation. The heat exchanger is generally classified into a heat exchanger provided inside a column and a heat exchanger provided outside the column, but the heat exchanger is not particularly limited in the present invention. The heat exchanger may be used as a vent gas condenser or a reboiler for heating a column bottom liquid in addition to as the condenser, and the number thereof installed is not particularly limited. A type of heat exchanger is not particularly limited, and specific examples of the heat exchanger include a vertical fixed tube plate type, a horizontal fixed tube plate sheet type, a U-tube type, a double-pipe type, a spiral type, a square block type, and a plate type.

The reflux tank is not particularly limited so long as the tank receives part or substantially the whole of the condensate flowing therein. A temperature of the liquid in the reflux tank is usually 15 to 40° C, due to a temperature of cooling water of the condenser used industrially. A temperature of the liquid in the reflux tank is preferably low because (meth)acrylic acid, which is an easily polymerizable compound, is handled in the reflux tank. An average residence time of the liquid in the reflux tank (liquid flow out rate / liquid residence volume) is usually 2 to 30 minutes, but is not particularly limited in the present invention.

In the present invention, the kind and amount of the above-mentioned polymerization inhibitor or polymerization retarder in the condensate in the reflux tank may be limited depending on an application of (meth) acrylic acid obtained in the present invention. The kind and amount thereof are not particularly limited when the application of (meth)acrylic acid obtained in the present invention is a raw material for acrylate or a raw material for high purity acrylate for super absorbent polymers, for example.

When the application of (meth)acrylic acid obtained in the present invention is a general acrylic acid product or a high purity acrylic acid product for example, methoxyhydroquinone is usually used as a polymerization inhibitor in a concentration of 180 to 220 ppm (standard of 200 ppm), as the custom. And following the custom is usually performed. Further, when the application of (meth)acrylic acid obtained in the present invention is an intermediate product, a concentration of the polymerization inhibitor is usually 10 to 200 ppm for the phenol compound and 5 to 100 ppm for the phenothiazine compound, though differing depending on the conditions such as an effect to a subsequent reaction.

A device used for supply of the oxygen-containing gas to the condensate is not particularly limited, but the oxygen-containing gas is preferably dispersed well in the condensate of (meth)acrylic acid. Based on such a standpoint, the oxygen-containing gas supplied into the liquid is preferably fined down by using, for example: fluid injection device for injecting a fluid in small particles such as a sparger having holes for injecting the fluid on a surface of a tube through which the oxygen-containing gas is introduced or a spray nozzle, or a porous body such as sintered metals; and an introduction tube for introducing the oxygen-containing gas to the fluid injection device.

Materials for various devices such as various nozzles , a column body, a reboiler, a condenser, a vent gas condenser, a reflux tank, a tube, and a pump as an attached device in each column used in the present invention are selected depending on the easily polymerizable compound used and temperature conditions. Stainless steels are often used as such materials, but the materials are not limited to stainless steels in the present invention. Examples of such materials include SUS 304, SUS 304L, SUS 316, SUS 316L, SUS 317, SUS 317L, SUS 327, and hastelloys. The materials are selected corresponding to physical properties of each liquid from a viewpoint of corrosion resistance.

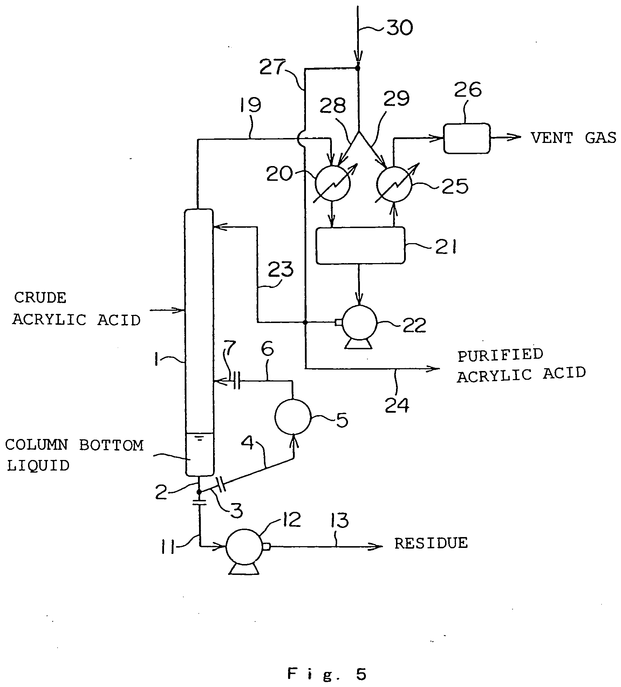

Hereinafter, an embodiment of the present invention will be described with reference to the drawings. Fig. 5 shows an example of a distillation apparatus for acrylic acid, which can carry out the present invention.

As shown in Fig. 5, the distillation apparatus for acrylic acid is provided with: a column body (distillation column) 1 for distilling crude acrylic acid; a condenser 20 for cooling a vapor containing acrylic acid for condensation thereof; a reflux tank 21 for receiving a condensate condensed in the condenser 20; a vent gas condenser 25 for further cooling a gas cooled in the condenser 20, to thereby recover valuable substances; and a vacuum equipment 26 for bringing a distillation system under reduced pressure.

A draw nozzle 2 for drawing a column bottom liquid is provided in a bottom part of the column body 1. An introduction nozzle 3 through which part of the drawn column bottom liquid is supplied and a tube 11 are connected to the draw nozzle 2.

A tube 4 for delivering the column bottom liquid from the introduction nozzle 3 is connected to the introduction nozzle 3, and a reboiler 5 for heating the column bottom liquid from the tube 4 is connected to the tube 4. A tube 6 for delivering the heated column bottom liquid is connected to the reboiler 5, and a nozzle 7 for supplying the column bottom liquid from the tube 6 to the column body 1 is connected to the tube 6.

A pump 12 for delivering the column bottom liquid from the tube 11 is connected to the tube 11, and a tube 13 is connected to the pump 12.

On the other hand, a tube 19 for delivering a gas containing acrylic acid is connected to a column top part of the column body 1. The condenser 20 is connected to the tube 19, and the reflux tank 21 is connected to the condenser 20. The vent gas condenser 25 and a pump 22 for delivering the condensate received in the reflux tank 21 are connected to the reflux tank 21. The vacuum equipment 26 is connected to the vent gas condenser 25.

A tube 23 for returning part of the condensate to the column body 1 is connected to the pump 22. From the tube 23, a tube 24 for delivering part of the condensate as purified acrylic acid and a tube 27 for delivering part of the condensate toward the condenser 20 and the vent gas condenser 25 branch.

A polymerization inhibitor supply tube 30 for supplying a polymerization inhibitor to part of the condensate is connected to the tube 27. From the tube 27, a first polymerization inhibitor supply tube 28 for supplying the condensate having the polymerization inhibitor supplied, to the condenser 20 and a second polymerization inhibitor supply tube 29 for supplying the condensate having the polymerization inhibitor supplied, to the vent gas condenser 25 branch. Further, a spray for spraying the polymerization inhibitor supplied from the first and second polymerization inhibitor supply tubes 28 and 29 into each of the condensers is provided in each of the condenser 20 and the vent gas condenser.

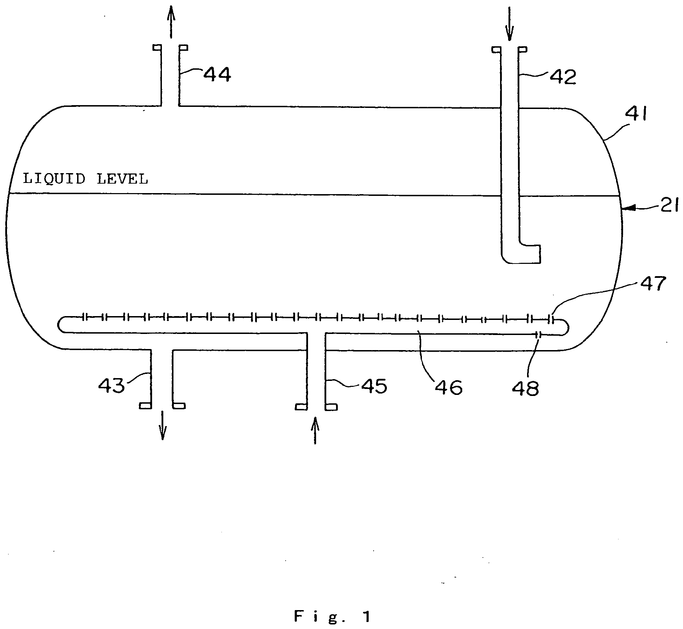

As shown in Fig. 1, the reflux tank 21 comprises: a tank body 41; an inlet nozzle 42 for introducing the condensate from the condenser 20 into the tank body 41; an outlet nozzle 43 for discharging the condensate received in the tank body 41 toward the pump 22; a gas outlet nozzle 44 for discharging a gas component in the tank body 41 toward the vent gas condenser 25; and a sparger (perforated tube) 46 provided in a lower part inside the tank body 41.

The sparger 46 is a tubular member which is provided substantially horizontally and comprises: a plurality of small holes 47 opened at a surface of an upper part of the sparger 46; and a liquid removal port 48 opened at a surface of a lower part of the sparger 46. An oxygen-containing gas introduction nozzle 45 for introducing the oxygen-containing gas into the sparger 46 from outside of the tank body 41 is connected to a lower part of the sparger 46.

A crude acrylic acid-containing liquid is introduced into the column body 1 for distillation, and part of the column bottom liquid circulates in an order of the draw nozzle 2, the introduction nozzle 3, the tube 4, the reboiler 5, the tube 6, and the nozzle 7. The other part of the column bottom liquid is taken out as a residue through the draw nozzle 2, the tube 11, the pump 12, and the tube 13.

A distillate from the column top is introduced to the condenser 20 through the tube 19 for condensation in the condenser 20, to thereby form a condensate of acrylic acid. On the other hand, a mixed liquid of the condensate of acrylic acid in the reflux tank 21 and the polymerization inhibitor supplied from the polymerization inhibitor supply tube 30 is sprayed into the condenser 20 from the spray. The condensate of acrylic acid condensed in the condenser 20 is introduced into the tank body 41 of the reflux tank 21 with the polymerization inhibitor from the condenser 20 through the inlet nozzle 42.

Part of the condensate of acrylic acid in the reflux tank 41 is returned to the column top through the pump 22 and the tube 23. Other part of the condensate of acrylic acid is supplied to the spray provided inside each of the condenser 20 and the vent gas condenser 25 through the first polymerization inhibitor supply tube 28 and the second polymerization inhibitor supply tube 29, and connected to the polymerization inhibitor supply tube 30 through the tube 27. The remaining condensate of acrylic acid is taken out as purified acrylic acid through the tube 24.

In the tank body 41, an oxygen-containing gas (air, for example) is supplied to the sparger 46 from the oxygen-containing gas introduction nozzle 45. The oxygen-containing gas is injected from the holes 47 and the liquid removal port 48 as bubbles each having a small particle size. The condensate of acrylic acid in the tank body 41 is subjected to gas-liquid contact with the oxygen-containing gas, thereby preventing polymerization of the condensate of acrylic acid. Thus, the polymerization of acrylic acid in the condensate in the reflux tank 21 and downstream thereof is prevented, thereby preventing formation of a polymerized product in tubes or pumps in the reflux tank 21 or downstream thereof.

A gas in the tank body 41 is cooled in the vent gas condenser 25 through the gas outlet nozzle 44. Acrylic acid which was condensed thereby is returned to the reflux tank 21 as a condensate, a gas component is taken out as a vent through the vacuum equipment 26.

In purification of acrylic acid in the distillation apparatus shown in Fig. 5, a column bottom temperature is preferably 60 to 120° C, particularly preferably 70 to 100° C. A column top pressure is preferably 1 to 50 kPa, particularly preferably 2 to 20 kPa.

In the present invention, an embodiment for supplying the oxygen-containing gas to the condensate in the reflux tank 21 is not limited to the embodiment shown in Fig. 1.

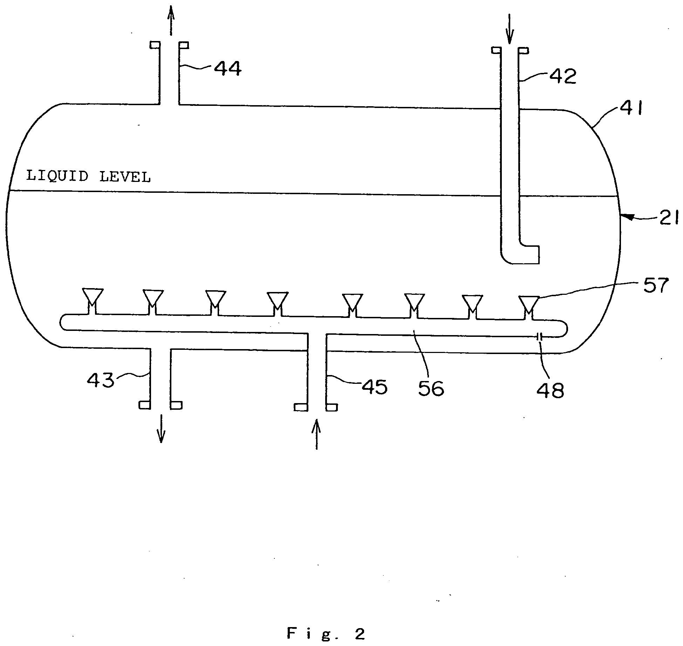

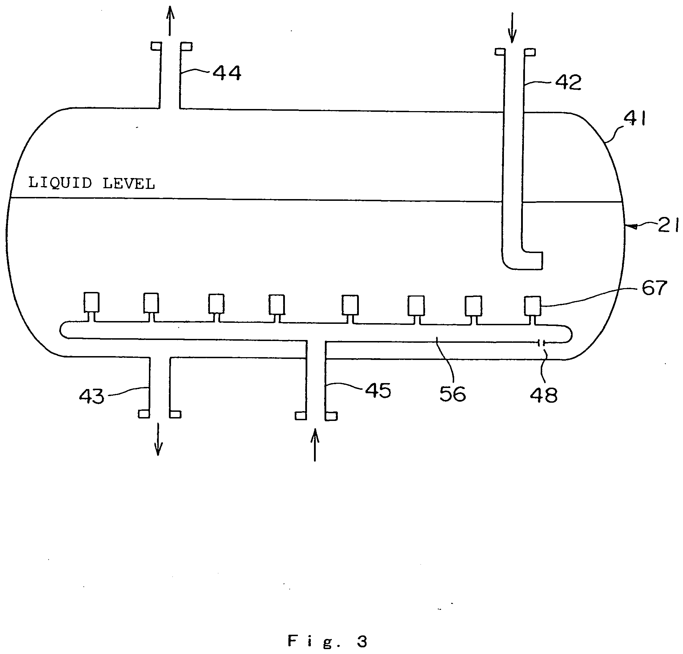

In the present invention, a reflux tank may comprise as shown in Fig. 2, for example: an introduction tube 56 which is a tubular member provided substantially horizontally in a lower part inside the tank body 41; and a plurality of sprays 57 provided above the introduction tube 56 for injecting a gas in the introduction tube 56 as fine bubbles, instead of the sparger 46. In the present invention, a reflux tank may comprise as shown in Fig. 3, for example, a plurality of sintered metals 67 which are porous bodies for discharging a gas in the introduction tube 56 as fine bubbles, instead of the sprays 57.

The embodiments shown in Figs. 2 and 3 can prevent polymerization of acrylic acid in the condensate in the reflux tank 21 or downstream thereof, thereby preventing formation of a polymerized product in tubes or pumps in the reflux tank 21 or downstream thereof as in the embodiment shown in Fig. 1.

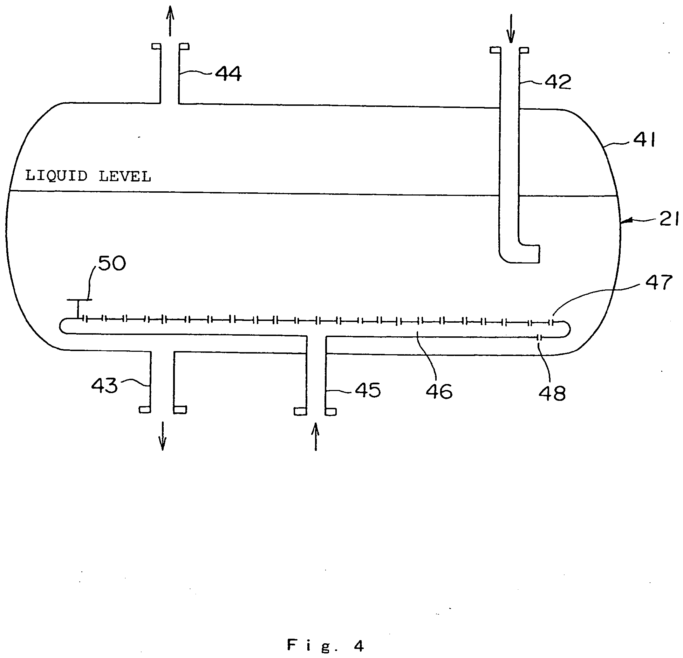

Further, part of a preferable embodiment of the present invention as shown in Fig. 4 for effective contact between the condensate of acrylic acid and the supplied oxygen-containing gas may further comprise a baffle board 50 provided inside the tank body 41 and above the sparger 46 for inhibiting elevation of bubbles injected from the holes 47. Such an embodiment allows a longer contact time between the condensate of acrylic acid in the tank body 41 and the oxygen-containing gas, which is even more effective for enhancing an effect of preventing polymerization of acrylic acid by the oxygen-containing gas.

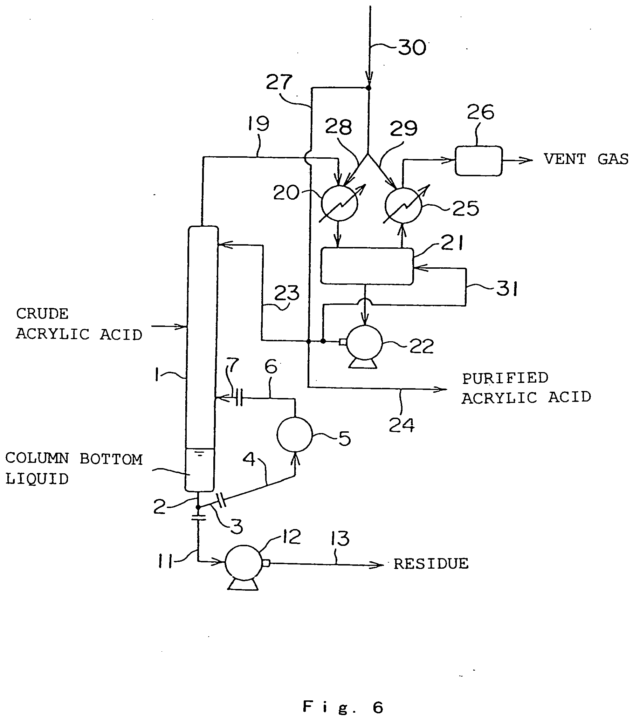

Further, another part of a preferable embodiment of the present invention as shown in Fig. 6 for more effective contact between the condensate of acrylic acid and the supplied oxygen-containing gas may further comprise a tube for circulation 31 which further branch from the tube 23 and connected to the reflux tank 21 for forcibly passing (circulating) the condensate of acrylic acid with the pump 22. Such an embodiment increases a concentration of the oxygen-containing gas in the condensate of acrylic acid, which is even more effective for enhancing the effect of preventing polymerization of acrylic acid by the oxygen-containing gas.

Examples

Next, the present invention will be described in more detail based on examples, but the present invention is not limited to the following examples without departing from the gist of the invention. Acrylic acid as a raw material and impurities were analyzed by using a gas chromatography device (GC14A, available from Shimadzu Corporation). Maleic acid converts into maleic anhydride during gas chromatography analysis and contents of both the maleic acid and the maleic anhydride cannot be specified. Thus, a total content of the maleic acid and the maleic anhydride will be referred to as a content of maleic acids in the following description.

<Example 1>

Distillation of a crude acrylic acid-containing liquid was carried out in the distillation apparatus shown in Fig. 5 using the distillation column of stainless steel SUS 316 having an inner diameter of 1, 100 mm, a length of 20,000 mm, and 21 perforated plates (dual flow trays) provided thereinside as the column body 1. A pump was provided in the middle of the tube 4 for supply of a column bottom liquid to the reboiler 5. The reboiler 5 was a vertical fixed tube plate-type heat exchanger, and the column bottom liquid flowed through tubes of the reboiler 5.

A mixture (supply liquid) containing 98.5 wt% acrylic acid, 0.3 wt% maleic acid, and 0.3 wt% acrylic acid dimer as the crude acrylic acid-containing liquid was supplied to the column body 1 at 90°C and 1,300 kg/h. Further, a liquid prepared by dissolving 8 wt% methoquinone and 1 wt% phenothiazine in acrylic acid was supplied to the supply liquid at 60 kg/h from a not-shown polymerization inhibitor-containing liquid tank.

An operation was carried out at a column top pressure of 2.8 kPa, a column bottom temperature of 75°C, a reflux ratio of 1, and a temperature of the condensate in the reflux tank 21 of 27° C, to thereby obtain high purity acrylic acid having a purity of 99.8 wt% or more from the column top. An amount of the acrylic acid taken out from the column top was 97 wt% with respect to the supply of the supply liquid to the column body 1.

A liquid prepared by dissolving 8 wt% methoquinone in acrylic acid was supplied at 3.08 kg/h from another not-shown polymerization inhibitor-containing liquid tank through the polymerization inhibitor supply tube 30.

The reflux tank 21 had an inner diameter of 820 mm and a length of 3,200 mm, and was provided with a sparger 46 (length of 2,500, hole diameter of 2 mm, 25 holes) inside and in a lower part thereof as shown in Fig. 1. Air as an oxygen-containing gas was supplied to the sparger 46 at 1.5 Nm³/h.

A continuous operation was carried out for 6 months. During the operation, the pump 22 which attached to the reflux tank 21 attained a continuous operation.

<Example 2>

An operation was carried out under the same conditions as those in Example 1 except that an introduction tube provided with sprays (1/8MJJRP015PTFE, available from H. Ikeuchi & Co., Ltd.) at 5 positions was used as shown in Fig. 2 instead of the sparger 46. A continuous operation was carried out for 6 months. During the operation, the pump 22 attained a continuous operation.

<Example 3>

An operation was carried out under the same conditions as those in Example 2 except that sintered metals (FUJIPLATE, available from FUJI FILTER MGF. CO., LTD.) having pores of 5 μm were used as shown in Fig. 3 instead of the sprays. A continuous operation was carried out for 6 months. During the operation, the pump 22 attained a continuous operation.

<Comparative Examples 1 to 3>

The supply of the oxygen-containing gas to the reflux tank 21 was stopped in Examples 1 to 3. 3 days after the stop, the pump 22 was stopped by overload. An inspection of the pump 22 confirmed a polymerized product between a shaft and a mechanical seal.

<Example 4>

A raw material for distillation was supplied at 1, 300 kg/h to a distillation column of stainless steel (SUS 316) having an inner diameter of 1,100 mm, a length of 20,000 mm, random packing (IMTP, available from Saint-Gobain NorPro) packed with 8 m inside thereof, and 9 perforated plates (dual flow trays) provided in a lower part thereof as the column body 1. A crude acrylic monomer as a raw material for distillation used was a mixture consisting of 98.5 wt% acrylic acid, 0.3 wt% maleic acid, 0.276 wt% acrylic acid dimer, 0.02 wt% furfural, and 0.004 wt% benzaldehyde.

Before supplying the mixture to the distillation column, with respect to the mixture, 1,650 ppm by weight hydrazine monohydrate, 40 ppm by weight copper dibutyldithiocarbamate, 40 ppm by weight copper acrylate, and 300 ppm by weight hydroquinone were mixed with the mixture. Copper acrylate used was prepared by dissolving cupric carbonate in acrylic acid, and the compounds and the mixing was performed at 20°C for 30 minutes.

An operation was carried out at a column top pressure of 10.1 kPa, a column bottom temperature of 95°C, a reflux ratio of 1, and a temperature of the condensate in the reflux tank 21 of 27° C, to thereby obtain high purity acrylic acid having a purity of 99.5 wt% or more and 1 ppm by weight or less of each of furfural and benzaldehyde from the column top. An amount of the acrylic acid taken out from the column top was 95 wt% with respect to the supply of the mixture to the column body 1.

The reflux tank 21 and the sparger 46 were the same as those in Example 1, and an air as an oxygen-containing gas was supplied to the sparger 46 at 2 Nm$^3$/h. Further, methoquinone was continuously added through the polymerization inhibitor supply tube 30 so that a methoquinone concentration in the liquid of acrylic acid in the reflux tank 21 fell within a range of 180 to 220 ppm by weight.

A continuous operation was carried out for 6 months. During the operation, the pump 22 attained a continuous operation.

<Example 5>

An operation was carried out under the same conditions as those in Example 4 except that an introduction tube provided with sprays at 5 positions was used as shown in Fig. 2 instead of the sparger. A continuous operation was carried out for 6 months. During the operation, the pump 22 attained a continuous operation.

<Example 6>

An operation was carried out under the same conditions as those in Example 5 except that sintered metals having pores of 3 μm were used as shown in Fig. 3 instead of the sprays. A continuous operation was carried out for 6 months. During the operation, the pump 22 attained a continuous operation.

<Comparative Examples 4 to 6>

The supply of the oxygen-containing gas to the reflux tank 21 was stopped in Examples 4 to 6. Several days after the stop, the pump 22 was stopped by overload. An inspection of the pump 22 confirmed a polymerized product between a shaft and a mechanical seal.

<Example 7>

An operation was carried out under the same conditions as those in Example 4 except that 2,200 ppm by weight of n-dodecylmercaptan as an aldehyde remover was mixed with the mixture instead of the hydrazine monohydrate in Example 4 with respect to the mixture, and a crude acrylic acid-containing liquid obtained by passing the mixture through a packed column of a sulfonic acid cation exchange resin (DIAION PK-216H, a trademark of Mitsubishi Chemical Corporation) was supplied to the distillation column in Example 4.

A continuous operation was carried out for 6 months. During the operation, the pump 22 attained a continuous operation.

Industrial Applicability

A method of purifying (meth)acrylic acid of the present invention is capable of preventing formation of a polymerized product from the obtained condensate of high purity (meth)acrylic acid and preventing clogging of a production line or abnormal operation of a production or purification apparatus due to the polymerized product, thereby allowing stable production of high purity (meth)acrylic acid over a long period of time.

Further, the present invention: solves the problems of overload in a pump which attaches to the reflux tank of the distillation column due to the polymerized product and difficulties in continuous operation for a long period of time when (meth)acrylic acid having a purity of 90% or more is obtained through distillation of crude (meth)acrylic acid obtained through vapor-phase catalytic oxidation; and allows stable production of high purity (meth)acrylic acid over a long period of time even if a product having higher purity is demanded in a future. Thus , the present invention has a significant industrial value.

**Claims**

1. A method of purifying (meth)acrylic acid, comprising the steps of:

   distilling a crude (meth)acrylic acid-containing liquid comprising acrylic acid or methacrylic acid to obtain a condensate of (meth)acrylic acid having a (meth)acrylic acid purity of 90% or more by a distillation apparatus provided with a reflux tank; and
   supplying an oxygen-containing gas containing oxygen to the condensate of (meth)acrylic acid in the reflux tank for receiving the condensate of (meth)acrylic acid.

2. The method of purifying (meth)acrylic acid according to claim 1, wherein the crude (meth)acrylic acid-containing liquid is obtained through a vapor-phase catalytic oxidation process.

3. The method of purifying (meth)acrylic acid according to claim 1 or 2, wherein the crude (meth)acrylic acid-containing liquid is distilled by using a distillation column in the distillation step.

4. The method of purifying (meth)acrylic acid according to any one of claims 1 to 3, wherein the condensate obtained in the distillation step has a (meth)acrylic acid purity of 95% or more.

5. The method of purifying (meth)acrylic acid according to any one of claims 1 to 4, wherein the oxygen-containing gas is a single gas of oxygen or air, or a mixed gas of the single gas and one or more gases selected from the group consisting of the following gases (1) to (4).

     (1) nitrogen
     (2) process exhaust gas
     (3) air (except when the single gas is air)
     (4) carbon dioxide

6. The method of purifying (meth)acrylic acid according to any one of claims 1 to 5, further comprising the step of adding a polymerization inhibitor to the condensate of (meth)acrylic acid.

7. The method of purifying (meth)acrylic acid according to claim 6, wherein the polymerization inhibitor is one or more compounds selected from the group consisting of copper (meth)acrylate, a copper dithiocarbamate, a phenol compound, and a phenothiazine compound.

8. The method of purifying (meth)acrylic acid according to claim 7, wherein the copper dithiocarbamate is one selected from the group consisting of copper dimethyldithiocarbamate, copper diethyldithiocarbamate, copper dipropyldithiocarbamate, copper dibutyldithiocarbamate, copper ethylenedithiocarbamate, copper tetramethylenedithiocarbamate, copper pentamethylenedithiocarbamate, copper hexamethylnedithiocarbamate, and copper oxydiethylenedithiocarbamate.

9. The method of purifying (meth)acrylic acid according to claim 7 or 8, wherein copper (meth)acrylate is prepared by dissolving one or more compounds selected from the group consisting of a copper powder, cupric carbonate, cuprous hydroxide, cupric hydroxide, and copper acetate in acrylic acid.

10. The method of purifying (meth)acrylic acid according to any one of claims 1 to 9, wherein the crude (meth)acrylic acid-containing liquid further contains a hydrazine compound.

11. The method of purifying (meth)acrylic acid according to claim 10, wherein the hydrazine compound is one selected from the group consisting of hydrazine, hydrazine hydrate, phenylhydrazine, hydrazine sulfate, and hydrazine chloride.

12. The method of purifying (meth)acrylic acid according to any one of claims 1 to 9, wherein the (meth)acrylic acid-containing liquid further contains a mercaptan.

13. The method of purifying (meth)acrylic acid according to claim 12, wherein the mercaptan is one selected from the group consisting of n-butyl mercaptan, n-octyl mercaptan, and n-dodecyl mercaptan.

14. The method of purifying (meth)acrylic acid according to any one of claims 1 to 13, wherein the crude (meth)acrylic acid-containing liquid is distilled in the presence of a phenol compound in the distillation step.

15. The method of purifying (meth)acrylic acid according to any one of claims 1 to 14, wherein the crude (meth)acrylic acid-containing liquid is distilled in the presence of a phenothiazine compound in the distillation step.

16. The method of purifying (meth)acrylic acid according to any one of claims 1 to 15, wherein the crude (meth)acrylic acid-containing liquid is distilled in the presence of copper (meth)acrylate in the distillation step.

17. The method of purifying (meth)acrylic acid according to any one of claims 1 to 16, wherein the crude (meth)acrylic acid-containing liquid is distilled in the presence of a copper dithiocarbamate in the distillation step.

**Patentansprüche**

1. Verfahren zur Reinigung von (Meth)acrylsäure, umfassend die Schritte:

Destillieren einer rohe (Meth)acrylsäure enthaltenden Flüssigkeit, umfassend Acrylsäure oder Methacrylsäure, um ein Kondensat von (Meth)acrylsäure mit einer (Meth)acrylsäure-Reinheit von 90 % oder mehr mittels einer mit einem Rückflussbehälter ausgestatteten Destillationsvorrichtung zu erhalten; und
Zuführen eines sauerstoffhaltigen Gases, enthaltend Sauerstoff, zu dem (Meth)acrylsäure-Kondensat in dem Rückflussbehälter, um das (Meth)acrylsäure-Kondensat zu erhalten.

2. Verfahren zur Reinigung von (Meth)acrylsäure gemäss Anspruch 1, wobei die rohe (Meth)acrylsäure enthaltende Flüssigkeit durch ein katalytisches Dampfphasenoxidationsverfahren erhalten wird.

3. Verfahren zur Reinigung von (Meth)acrylsäure gemäss Anspruch 1 oder 2, wobei die rohe (Meth)acrylsäure enthaltende Flüssigkeit unter Verwendung einer Destillationskolonne im Destillationsschritt destilliert wird.

4. Verfahren zur Reinigung von (Meth)acrylsäure gemäss irgendeinem der Ansprüche 1 bis 3, wobei das im Destillationsschritt erhaltende Kondensat eine (Meth)acrylsäure-Reinheit von 95 % oder mehr aufweist.

5. Verfahren zur Reinigung von (Meth)acrylsäure gemäss irgendeinem der Ansprüche 1 bis 4, wobei das sauerstoffhaltige Gas ein einzelnes Gas aus Sauerstoff oder Luft oder ein Gasgemisch aus dem einzelnen Gas und einem oder mehreren Gasen, ausgewählt aus der Gruppe bestehend aus den nachstehenden Gasen (1) bis (4) ist:

   (1) Stickstoff
   (2) Prozessabgas
   (3) Luft (ausser wenn das einzelne Gas Luft ist)
   (4) Kohlendioxid.

6. Verfahren zur Reinigung von (Meth)acrylsäure gemäss irgendeinem der Ansprüche 1 bis 5, das ferner einen Schritt umfasst, in dem ein Polymerisationsinhibitor zu dem (Meth)acrylsäure-Kondensat zugegeben wird.

7. Verfahren zur Reinigung von (Meth)acrylsäure gemäss Anspruch 6, wobei der Polymerisationsinhibitor eine oder mehrere Verbindung(en), ausgewählt aus der Gruppe bestehend aus Kupfer(meth)acrylat, einem Kupferdithiocarbamat, einer Phenolverbindung und einer Phenothiazinverbindung, ist.

8. Verfahren zur Reinigung von (Meth)acrylsäure gemäss Anspruch 7, wobei das Kupferdithiocarbamat eines, ausgewählt aus der Gruppe bestehend aus Kupferdimethyldithiocarbamat, Kupferdiethyldithiocarbamat, Kupferdipropyldithiocarbamat, Kupferdibutyldithiocarbamat, Kupferethylendithiocarbamat, Kupfertetramethylendithiocarbamat, Kupferpentamethylendithiocarbamat, Kupferhexamethylendithiocarbamat und Kupferoxydiethylendithiocarbamat, ist.

9. Verfahren zur Reinigung von (Meth)acrylsäure gemäss Anspruch 7 oder 8, wobei das Kupfer(meth)acrylat durch Lösen von einer oder mehreren Verbindungen, ausgewählt aus der Gruppe bestehend aus einem Kupferpulver, Kupfercarbonat, Kupferhydroxid, Kupfer(II)hydroxid und Kupferacetat, in Acrylsäure hergestellt ist.

10. Verfahren zur Reinigung von (Meth)acrylsäure gemäss irgendeinem der Ansprüche 1 bis 9, wobei die rohe (Meth)acrylsäure enthaltende Flüssigkeit ferner eine Hydrazinverbindung enthält.

11. Verfahren zur Reinigung von (Meth)acrylsäure gemäss Anspruch 10, wobei die Hydrazinverbindung eine, ausgewählt aus der Gruppe bestehend aus Hydrazin, Hydrazinhydrat, Phenylhydrazin, Hydrazinsulfat und Hydrazinchlorid, ist.

12. Verfahren zur Reinigung von (Meth)acrylsäure gemäss irgendeinem der Ansprüche 1 bis 9, wobei die (Meth)acrylsäure enthaltende Flüssigkeit ferner ein Mercaptan enthält.

13. Verfahren zur Reinigung von (Meth)acrylsäure gemäss Anspruch 12, wobei das Mercaptan eines, ausgewählt aus der Gruppe bestehend aus n-Butylmercaptan, n-Octylmercaptan und n-Dodecylmercaptan, ist.

14. Verfahren zur Reinigung von (Meth)acrylsäure gemäss irgendeinem der Ansprüche 1 bis 13, wobei die rohe (Meth)acrylsäure enthaltende Flüssigkeit in Gegenwart einer Phenolverbindung im Destillationsschritt destilliert wird.

15. Verfahren zur Reinigung von (Meth)acrylsäure gemäss irgendeinem der Ansprüche 1 bis 14, wobei die rohe (Meth)acrylsäure enthaltende Flüssigkeit in Gegenwart einer Phenothiazinverbindung im Destillationsschritt destilliert wird.

16. Verfahren zur Reinigung von (Meth)acrylsäure gemäss irgendeinem der Ansprüche 1 bis 15, wobei die rohe (Meth)acrylsäure enthaltende Flüssigkeit in Gegenwart von Kupfer(meth)acrylat im Destillationsschritt destilliert wird.

17. Verfahren zur Reinigung von (Meth)acrylsäure gemäss irgendeinem der Ansprüche 1 bis 16, wobei die rohe (Meth)acrylsäure enthaltende Flüssigkeit in Gegenwart eines Kupferdithiocarbamats im Destillationsschritt destilliert wird.

**Revendications**

1. Procédé de purification d'acide (méth)acrylique, comprenant les étapes de :

   distillation d'un liquide contenant de l'acide (méth)acrylique brut comprenant de l'acide acrylique ou de l'acide méthacrylique pour obtenir un condensat d'acide (méth)acrylique ayant une pureté d'acide (méth)acrylique de 90 % ou plus par un appareil de distillation pourvu d'une cuve à reflux; et
   fourniture d'un gaz contenant de l'oxygène qui contient de l'oxygène au condensat d'acide (méth)acrylique dans la cuve à reflux pour recevoir le condensat d'acide (méth)acrylique.

**2.** Procédé de purification d'acide (méth)acrylique selon la revendication 1, dans lequel le liquide contenant de l'acide (méth)acrylique brut est obtenu par un processus d'oxydation catalytique en phase vapeur.

**3.** Procédé de purification d'acide (méth)acrylique selon la revendication 1 ou 2, dans lequel le liquide contenant de l'acide (méth)acrylique brut est distillé en utilisant une colonne de distillation dans l'étape de distillation.

**4.** Procédé de purification d'acide (méth)acrylique selon l'une quelconque des revendications 1 à 3, dans lequel le condensat obtenu dans l'étape d'oxydation a une pureté d'acide (méth)acrylique de 95 % ou plus.

**5.** Procédé de purification d'acide (méth)acrylique selon l'une quelconque des revendications 1 à 4, dans lequel le gaz contenant de l'oxygène est un gaz unique d'oxygène ou d'air, ou un gaz mixte du gaz unique et d'un ou plusieurs gaz choisis dans le groupe consistant en les gaz (1) à (4) suivants :

    (1) l'azote
    (2) un gaz d'échappement de traitement
    (3) l'air (à l'exception du cas où le gaz unique est de l'air)
    (4) le dioxyde de carbone.

**6.** Procédé de purification d'acide (méth)acrylique selon l'une quelconque des revendications 1 à 5, comprenant en outre l'étape d'addition d'un inhibiteur de polymérisation au condensat d'acide (méth)acrylique.

**7.** Procédé de purification d'acide (méth)acrylique selon la revendication 6, dans lequel l'inhibiteur de polymérisation est un ou plusieurs composés choisis dans le groupe consistant en le (méth)acrylate de cuivre, un dithiocarbamate de cuivre, un composé de phénol, et un composé de phénothiazine.

**8.** Procédé de purification d'acide (méth)acrylique selon la revendication 7, dans lequel le dithiocarbamate de cuivre est un élément choisi dans le groupe consistant en le diméthyldithiocarbamate de cuivre, le diéthyldithiocarbamate de cuivre, le dipropyldithiocarbamate de cuivre, le dibutyldithiocarbamate de cuivre, l'éthylènedithiocarbamate de cuivre, le tétraméthylènedithiocarbamate de cuivre, le pentaméthylènedithiocarbamate de cuivre, l'hexaméthylènedithiocarbamate de cuivre, et l'oxydiéthylènedithiocarbamate de cuivre.

**9.** Procédé de purification d'acide (méth)acrylique selon la revendication 7 ou 8, dans lequel du (méth)acrylate de cuivre est préparé par dissolution d'un ou plusieurs composés choisis dans le groupe consistant en une poudre de cuivre, du carbonate cuivrique, de l'hydroxyde cuivreux, de l'hydroxyde cuivrique, et de l'acétate de cuivre dans de l'acide acrylique.

**10.** Procédé de purification d'acide (méth)acrylique selon l'une quelconque des revendications 1 à 9, dans lequel le liquide contenant de l'acide (méth)acrylique brut contient en outre un composé d'hydrazine.

**11.** Procédé de purification d'acide (méth)acrylique selon la revendication 10, dans lequel le composé d'hydrazine est un élément choisi dans le groupe consistant en l'hydrazine, l'hydrate d'hydrazine, la phénylhydrazine, le sulfate d'hydrazine et le chlorure d'hydrazine.

**12.** Procédé de purification d'acide (méth)acrylique selon l'une quelconque des revendications 1 à 9, dans lequel le liquide contenant de l'acide (méth)acrylique contient en outre un mercaptan.

**13.** Procédé de purification d'acide (méth)acrylique selon la revendication 12, dans lequel le mercaptan est un choisi dans le groupe consistant en le n-butyl mercaptan, le n-octyl mercaptan et le n-dodécyl mercaptan.

**14.** Procédé de purification d'acide (méth)acrylique selon l'une quelconque des revendications 1 à 13, dans lequel le liquide contenant de l'acide (méth)acrylique brut est distillé en présence d'un composé de phénol dans l'étape de distillation.

**15.** Procédé de purification d'acide (méth)acrylique selon l'une quelconque des revendications 1 à 14, dans lequel le liquide contenant de l'acide (méth)acrylique brut est distillé en présence d'un composé de phénothiazine dans l'étape de distillation.

**16.** Procédé de purification d'acide (méth)acrylique selon l'une quelconque des revendications 1 à 15, dans lequel le liquide contenant de l'acide (méth)acrylique brut est distillé en présence de (méth)acrylate de cuivre dans l'étape de distillation.

**17.** Procédé de purification d'acide (méth)acrylique selon l'une quelconque des revendications 1 à 16, dans lequel le liquide contenant de l'acide (méth)acrylique brut est distillé en présence d'un dithiocarbamate de cuivre dans l'étape de distillation.

F i g . 1

LIQUID LEVEL

F i g. 2

LIQUID LEVEL

Fig. 3

LIQUID LEVEL

44

42

41

21

50

47

43

45 46 48

F i g . 4

F i g. 5

CRUDE
ACRYLIC ACID →

COLUMN BOTTOM
LIQUID

VENT GAS

PURIFIED
ACRYLIC ACID

RESIDUE

F i g. 6

**EP 1 688 407 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 49030312 A
- JP 58037290 B
- JP 7330659 A
- JP 2001316326 A
- JP 7228548 A
- JP 2001122909 A
- JP 49085016 A
- JP 2001058970 A
- JP 2001213839 A